# EUROPEAN PATENT SPECIFICATION

(11) **EP 1 241 959 B1**
(45) Date of publication and mention of the grant of the patent: **11.09.2013**
(21) Application number: 00988176.4
(22) Date of filing: 18.12.2000
(51) Int. Cl.: A61K 8/81, A61K 8/87, A61K 8/92, A61Q 3/02

(54) **Use of soak-off radiation curable nail coatings**
Verwendung von aufweichbaren strahlungshärtbaren Nagellacken
Utilisation des vernis à ongles décollants durcissant par rayonnement

(30) Priority: 17.12.1999 US 466985; 17.12.1999 US 466986; 17.12.1999 US 467127
(43) Date of publication of application: 25.09.2002
(73) Proprietor: Creative Nail Design, Inc., Vista, CA 92083 (US)
(72) Inventor: LILLEY, Pamela, H., Prescott, AZ 86305 (US); PETERSON, Sarah, Laurel, DE 19956 (US)
(74) Representative: Van Breda, Jacobus
(86) International application number: PCT/US2000/034555
(87) International publication number: WO 2001/043579

(56) References cited:
- EP-A- 0 453 628
- GB-A- 656 264
- KR-B1- 97 002 606
- US-A- 2 979 061
- US-A- 4 415 903
- US-A- 4 600 030
- US-A- 4 666 952
- US-A- 4 682 612
- US-A- 4 690 369
- US-A- 4 721 735
- US-A- 4 745 003
- US-A- 4 766 005
- US-A- 4 844 102
- US-A- 4 867 680
- US-A- 5 071 888
- US-A- 5 453 451
- US-A- 5 708 052
- US-A- 5 792 447
- US-A- 5 965 111
- US-A- 5 985 951

## Description

### CROSS-REFERENCE TO RELATED APPLICATIONS

This application claims the benefit of the filing of and is a continuation-in-part application of U.S. Patent Application Serial No. 09/466,985, entitled "Pre-Bond Compounds For Radiation Curable Nail Coatings", to Pamela H. Lilley, filed on December 17, 1999 ('985 Application); U.S. Patent Application Serial No. 09/466,986, entitled "Radiation Curable Nail Coatings", to Pamela H. Lilley, filed on December 17, 1999 ('986 Application); and U.S. Patent Application Serial No. 09/467,127, entitled "Finishing Compounds for Radiation Curable Nail Coatings", to Pamela H. Lilley, filed on December 17, 1999 ('127 Application).

### BACKGROUND OF THE INVENTION

### Field of the invention (Technical Field):

The present Invention relates to radiation curable nail treatment materials and methods of using the same.

### Background Art:

Note that the following discussion refers to a number of publications by author(s) and year of publication, and that due to recent publication dates certain publications are not to be considered as prior art vis-a-vis the present invention. Discussion of such publications herein is given for more complete background and is not to be construed as an admission that such publications are prior art for patentability determination purposes.

Light curable nail coatings are disclosed in Billings, U.S. Patent No. 5,194,292, entitled "Method of Drying and Bonding Nail Polish" ('292 Patent); Cornell, U.S. Patent No. 4,704,303, entitled "Nail Extension Composition" ('303 Patent); and Gulliano, U.S. Patent No. 4,682,612, entitled "Novel Process and Article for Preparing Artificial Nails" ('612 Patent). The '292 Patent describes a method of protecting common nail polish by applying a light-curable clear coating over the polished nail. The '303 Patent describes a coating composition based on an aliphatic or cycloaliphatic hydrocarbon urethane diacrylate or (meth)acrylate having a molecular weight of 250 to 500 and a viscosity of 5,000 to 30,000 cps. Radiation in the visible region is used to cure the coatings disclosed in the '303 Patent. The '612 Patent describes an organic solvent-free photocurable composition that has at least one liquid monomer in which an acrylated urethane oligomer is dissolved and crosslinked upon curing, Radiation in the ultraviolet (UV) region is used to cure the coatings disclosed in the '612 Patent. None of these patents disclose the use of Bisphenol A Diglycidyl (Meth)Acrylate ("BISGMA") based urethane resin.

US4260701 (A) recties improved composition for a fingernail coating having an acrylic binder, a peroxide catalyst, a tertiary amine accelerator, and a polymeric filler at least partially soluble in the coating. The acrylic binder contains a monoethylenically unsaturated monomer comprising at least a major proportion of methoxyethyl methacrylate. A polyfunctional monomer may be present that copolymerizes with the monoethylenically unsaturated monomer, for crosslinking and toughening.

EP0453628 (A2) recites synthetic coatings which adhere well to fingernails can be produced with photopolymerisable fingernail products based on methacrylate, which contain 1-20% by weight of at least one monomethacryloyloxyethyl ester of a dicarboxylic acid.

US474S003 (A) recites a method of coating the metal side of a mirror comprising applying to a reflective layer of metal adhered to a transparent glass sheet one or more adherent protective layers wherein the outermost protective layer is a cured coating formed from a liquid coating composition which is curable by exposure to ultraviolet light (UV). It ts discovered that it is important to the method that the UV curable liquid coating composition have a high degree of resistance to shrinkage upon curing. It is important that the UV curable liquid coating composition not exhibit a "Percent Shrinkage" of 10 percent or more as defined below.

Problems associated with traditional light curable nail coatings include a tendency for the coating to run on the nail during application due to low viscosity. Consequently, coatings tend to run onto the cuticle or other unwanted areas and/or cause lifting of the coating over time. In contrast, nail treatment materials of the present invention optionally comprise a creamy consistency with a viscosity of between approximately 20,000-80,000 cps. The high viscosity of such materials allows for brushing onto a nail and/or a nail tip without significant running and/or spreading during application, which, In turn, generally reduces any subsequent lifting.

Another problem with traditional light curable nail coatings is associated with the use of urethane resins. Often urethane resins are made with high levels of toxic catalysts, which pose a significant risk of skin sensitization. In contrast, urethane resins of an embodiment of the present invention require relatively low levels of catalyst, and thus do not generally cause skin sensitization in the general population.

Another problem with traditional light curable nail coatings is that over time the coatings tend to lift from the natural nail. The present invention overcomes this problem through the optional application of pre-bond materials that enhance the bond between the natural nail and inventive coatings of the present invention, or alternatively, traditional light curable nail coatings.

Another problem with traditional light curable nail coatings is that, upon curing, the surface of the coating becomes sticky and rough due to an air diffused layer, wherein the air inhibits curing. Generally, ethyl alcohol is applied to a coating surface to remove the undesirable air diffused layer. The present invention includes various materials that improve the final appearance and characteristics of coated nails.

Traditional nail coating materials generally include two varieties: polish type, which cure by solvent evaporation, and polymer type, which cure by chemical reaction. If a wearer desires a more natural look, and has strong nails, a polish type material is usually chosen to enhance appearance and add protection. If a wearer has short, weak nails, and desires longer nail enhancements, then a polymer type material is suitable. Polymer type materials include, for example, powder/liquid systems and gel systems. Gel systems, particularly ultraviolet cured gel systems, often comprise a gel that is brushed onto the nails, cured, and shaped to create life like artificial nails. In contrast to powder/liquid systems, gel systems are relatively easy to use, applicable in less time, lightweight on the nail, have no odor or only minimal odor, are durable, and have a high quality shine.

Polish drying by solvent evaporation is one of few traditional methods that avoid etching of the nail surface. Another method that avoids etching includes the use of "wraps." Overall, a need exists for a material, colored or not, which is easily applied, dries rapidly, protects the nail more than polish, and can be removed when the wearer desires. Such a material is described in an embodiment of the present invention. In addition, for wearers of artificial nail enhancements, there is a need for a coating, which dries rapidly (almost immediately), doesn't chip, and can be removed at a later date for versatility. The radiation curable coatings of various embodiments of the present invention, and aforementioned related disclosures, satisfy such a need. The inventive methods of embodiments of the present invention also help to address this need.

### SUMMARY OF THE INVENTION (DISCLOSURE OF THE INVENTION)

The present invention encompasses uses and methods according to claims 1-8. There are compositions comprising: at least one solvent; a hydrogenated rosin; and a (meth)acrylate resin; the composition to be applied to a natural nail, hoof, or claw to enhance a bond between the natural nail, hoof, or claw and a nail coating or a polymeric filler and wherein the rosin optionally comprises a glycerol ester and wherein the composition optionally comprises between approximately 2 percent and approximately 30 percent by weight rosin and wherein the (meth)acrylate resin optionally comprises at least one type of (meth)acrylate resin and wherein the composition optionally comprises between approximately 2 percent and approximately 30 percent by weight (meth)acrylate resin and wherein the (meth)acrylate resin optionally comprises a (meth)acrylate urethane resin.

There are methods to enhance the bond between a natural nail, hoof, or claw and a nail coating or polymeric filler compound, the method comprising the steps of: cleaning the surface to be coated or filled; applying a pre-bond compound to the surface wherein the pre-bond compound comprises at least one solvent, a hydrogenated rosin and a (meth)acrylate resin; permitting solvent to at least partially evaporate; and applying a polymerizable coating or filler to the at least partially evaporated pre-bond compound.

There are compositions comprising: a polymerizable compound; a photoinitiator; and a photoaccelerator, the composition to be applied to natural nails and artificial nail tips, and wherein the composition optionally comprises a coupling agent and wherein the composition optionally comprises at least one additive selected from the group consisting of plasticizers, secondary photoinitiators, colorants, dyes, inhibitors, fillers, fibers, and adhesion promoting polymers and wherein the composition optionally comprises at least one polymerizable (meth)acrylate resin and wherein the (meth)acrylate resin optionally comprises a (meth)acrylate urethane resin and wherein the photoinitiator optionally comprises at least one initiator selected from the group consisting of phosphinates and phosphine oxides and sulfanyl ketone and wherein the photoaccelerator optionally comprises at least one accelerator selected from the group consisting of aliphatic amines and aromatic amines and wherein the coupling agent optionally comprises titanate.

There are compositions comprising: a polymerizable BISGMA urethane resin; a polymerizable (meth)acrylate resin; a photoinitiator; and a photoaccelerator, the composition to be applied to natural nails and artificial nail tips and wherein the composition optionally comprises a coupling agent and wherein the composition optionally comprises at least one additive selected from the group consisting of plasticizers, colorants, dyes, inhibitors, fillers, fibers, adhesion promoting monomers or polymers, and crosslinking monomers or polymers and wherein the composition optionally comprises between approximately 0.1 percent and 95 percent by weight BISGMA urethane and wherein the (meth)acrylate resin optionally comprises a polyol modified (meth)acrylate resin and wherein the polyol optionally comprises at least one polyol selected from the group consisting of polyether, polyester, and polycarbonate and wherein the (meth)acrylate resin optionally comprises a (meth)acrylate urethane resin.

There are compositions comprising: a polymerizable urethane dimethacrylate resin; a polymerizable (meth)acrylate resin; a photoinitiator; and a photoaccelerator, the composition to be applied to natural nails and artificial nail tips and wherein the composition optionally comprises a coupling agent and wherein the composition optionally comprises at least one additive selected from the group consisting of plasticizers, colorants, dyes, inhibitors, fillers, fibers, adhesion promoting monomers or polymers, and crosslinking monomers or polymers and wherein the composition optionally comprises between approximately 0.1 percent and 95 percent by weight urethane dimethacrylate resin and wherein the (meth)acrylate optionally comprises a polyol modified (meth)acrylate and wherein the polyol optionally comprises at least one polyol selected from the group consisting of polyether, polyester, and polycarbonate and wherein the (meth)acrylate resin optionally comprises a (meth)acrylate urethane resin.

There are compositions comprising: at least one solvent; and a plasticizer, the composition to be applied to cured polymeric or urethane nail coatings and wherein the at least one solvent optionally comprises at least one solvent selected from the group consisting of acetone, ethyl alcohol, ethyl acetate, isopropyl alcohol, and methyl ethyl ketone and wherein the plasticizer optionally comprises at least one plasticizer selected from the group consisting of phthalates, adipates, and sulfonamides and wherein the composition optionally comprises at least one vegetable oil and wherein the composition optionally comprises lanolin and wherein the at least one vegetable oil comprises castor oil.

There are methods of removing an air-inhibited layer from a radiation cured coating, the method comprising the steps of: providing a radiation cured coating; applying a composition to the radiation cured coating, the composition comprising at least one solvent and a plasticizer and wherein the at least one solvent optionally comprises at least one solvent selected from the group consisting of acetone, ethyl alcohol, ethyl acetate, isopropyl alcohol, and methyl ethyl ketone and wherein the plasticizer optionally comprises at least one plasticizer selected from the group consisting of phthalates, adipates, and sulfonamides and wherein the composition optionally comprises at least one vegetable oil and wherein the composition optionally comprises lanolin.

There is described a method of reducing soak-off characteristics of a nail coating composition, the method comprising the step of adding a polymerizable BISGMA urethane resin to the composition and wherein the composition optionally comprises a polymerizable (meth)acrylate resin and wherein the (meth)acrylate resin optionally comprises at least two different molecular weight (meth)acrylate resins and wherein the (meth)acrylate resin optionally comprises a polyol modified (meth)acrylate resin and wherein the (meth)acrylate resin optionally comprises a (meth)acrylate urethane resin and wherein adding the BISGMA urethane resin to the nail coating composition forms a final composition, the final composition optionally comprising between approximately 0.1 percent and approximately 99 percent by weight of the BISGMA urethane resin.

There is a method of increasing the hardness of a cured nail coating composition, the method comprising the step of adding a polymerizable BISGMA urethane resin to the nail coating composition prior to curing and wherein the composition optionally comprises a polymerizable (meth)acrylate resin and wherein the (meth)acrylate resin optionally comprises at least two different molecular weight (meth)acrylate resins and wherein the (meth)acrylate resin optionally comprises a polyol modified (meth)acrylate resin and wherein the (meth)acrylate resin optionally comprises a (meth)acrylate urethane resin and wherein adding the BISGMA urethane resin to the nail coating composition forms a final composition, the final composition optionally comprising between approximately 0.1 percent and approximately 99 percent by weight of the BISGMA urethane resin.

In one embodiment, the present invention comprises a method of applying a soak-off nail coating composition to a coated nail, the method comprising the steps of: providing a coated nail; applying a composition comprising a polymerizable polyol modified (meth)acrylate resin to the coated nail wherein the (meth)acrylate resin optionally comprises a (meth)acrylate urethane resin; and curing the applied composition.

In another embodiment, the present invention comprises a method of removing a soak-off nail coating composition from a coated nail, the method comprising the steps of: providing a nail coated with a radiation cured composition, the cured composition comprising a polyol modified (meth)acrylate polymer wherein the (meth)acrylate resin optionally comprises a (meth)acrylate urethane resin; and soaking the coated nail with a solvent, wherein the solvent comprises a polar solvent.

A nail coating can be prepared comprising: polymerized BISGMA urethane resin; and polymerized (meth)acrylate resin wherein the (meth)acrylate resin optionally comprises a (meth)acrylate urethane resin and wherein the (meth)acrylate resin optionally comprises a polyol modified (meth)acrylate resin.

It is desirable to provide hard and durable coatings for the cosmetic industry, particularly for the cosmetic nail industry.

It is desirable to provide high gloss and smooth finishing materials that are applied to the coated fingernails after curing.

It is desirable to provide pre-bonding materials that are applied to a natural nail to enhance bonding between a nail coating and the natural nail.

It is desirable that the coating materials are of sufficient viscosity and/or other rheological properties such that the materials do not tend to run off the nail and onto the finger or toe, etc.

It is desirable that the coating materials result in a strong and durable bond to both artificial nail tips and natural nails.

It is desirable that the coating materials comprise chemical materials that exhibit a low degree of skin sensitivity.

It is desirable that a pre-bonding material may be used in conjunction with the coating materials.

It is desirable that a finishing material may be used In conjunction with the coating materials to clean the surface of the coating and apply a polished, high gloss surface.

### DESCRIPTION OF THE PREFERRED EMBODIMENTS

### (BEST MODES FOR CARRYING OUT THE INVENTION)

The stiffness of radiation curable nail coatings, In their cured state, varies depending on polymer composition and polymerization initiation. It is recognized that coating removal is also related to composition and method of initiation. Formulas in the present invention allow for a wide range of radiation curable nail coating materials that meet the stiffness and removal needs of most all wearers of artificial nail products. Some of the materials in the present description are optionally sculptable to form an artificial nail and, once applied, are preferably removable through filing rather than through soaking. Other materials in the present description are preferably removable through soaking rather than through filing.

The radiation curable coatings in the present description are photochemistry described below and in aforementioned related applications, based embodiments, compositions or materials of the present invention comprise a photochemical system of phosphinate, amine functional compound, neoalkoxy titanate, and/or a secondary photoinitiator. Throughout this disclosure, the term "material," in singular and/or plural may substitute for composition, e.g., composition of matter.

While the compositions in the present invention do not exclude the use of acrylates, methacrylates are preferred because methacrylates are less likely to cause skin sensitization than acrylate formulas. The term "(meth)acrylate" as used herein, means methacrylate, acrylate, or mixtures thereof.

Ranges for BISGMA urethane resin and (meth)acrylated polyether urethane disclosed herein and in prior, related patent applications are, in some embodiments, suitable for supporting a material that serves to permanently extend a natural nail. Once applied, such a material is removed by filing rather than by soaking off in a solvent, such as, acetone.

Soak-off materials in the present invention comprise resins. The term "soak-off" refers to the ability to remove a material from a nail with a solvent-based solution, typically in conjunction with a "wrap," for example, but not limited to, an aluminum foil wrap. According to the present invention, it is possible to tailor the characteristics of a soak-off material by varying resin type and resin concentration.

The concentration of (meth)acrylated polyether urethane resin In a soakoff material may be higher than in a comparable permanent, non-soak-off material. Alternatively the concentration of BISGMA urethane resin in a soak-off material may be lower than in permanent, non-soak-off material. According to the present description, adjustable parameters include, but are not limited to, (I) blend ratio of BISGMA urethane resin to (meth)acrylated polyether urethane and (ii) molecular weight of (meth)acrylated polyether urethane, which optionally comprise custom blends of different molecular weight (meth)acrylated polyether urethanes.

A permanent type resin can be altered through use of (meth)acrylated polyether urethane resins to give desirable soak-off characteristics.

In a variety of embodiments, soak-off materials give rise to a new option for wearers who desire: 1) color on their natural nails; 2) protection for new growth; 3) rapid drying to a smudgeproof surface; and/or 4) versatility to easily change color. A variety of soak-off materials of the present invention are also optionally useful on top of nail enhancements. For example, see "Method of Creating a Permanent Nail Enhancement," which provides for a non-chipping color with rapid drying to a smudgeproof surface.

### Pre-bond Materials of the '985 Application

The following embodiments are disclosed in the '985 Application. A pre-bond material is described for application to a natural nail to enhance bonding between the natural nail and a nail coating. The pre-bond material comprises at least one solvent and a (meth)acrylate oligomer. The pre-bond material optionally comprises: between approximately 50 and 95 percent by weight, preferably between approximately 60 and 80 percent by weight, of solvent; and between approximately 2 and 30 percent by weight, preferably between approximately 5 and 20 percent by weight, of (meth)acrylate oligomer. In one embodiment, the pre-bond material optionally comprises: at least one solvent such as, alcohols, ketones, and/or esters, and the (meth)acrylate oligomer comprises methacrylate, preferably an acid methacrylate. In another embodiment, the solvent comprises acetone, and the (meth)acrylate oligomer comprises the acid methacrylate oligomer commercially formulated as SARBOX® SB 500E50 (Sartomer Co., Inc., Exton, Pennsylvania). This particular (meth)acrylate (SB 500E50) is a highly functional, carboxylic acid and anhydride containing methacrylate oligomer blended in SR-454, ethoxylated trimethylolpropane triacrylate monomer. Further, the pre-bond material optionally comprises hydrogenated rosin, preferably hydrogenated rosin of a glycerol ester. One such rosin is commercially formulated as FORMAL® 85 (Hercules, Wilmington, Delaware) synthetic wood rosin derived resin (comprising, for example, but not limited to, abietic acid). The pre-bond material optionally comprises between approximately 2 and 30 percent by weight, preferably between approximately 5 and 20 percent by weight of hydrogenated rosin.

The description also comprises a method to enhance the bond between a natural nail, hoof, or claw and a nail coating or polymeric filler material, for example, but not limited to, a radiation curable filler. In one embodiment, a first embodiment, the bond method comprises the steps of: cleaning the surface to be coated or filled; applying a pre-bond material comprising at least one solvent and a (meth)acrylate oligomer; and permitting the solvent to partially evaporate. The polymeric coating or filler is then applied to the partially solvent evaporated pre-bond material. In another embodiment, a second embodiment, the bond method used is similar to the aforementioned embodiment with the exception that the pre-bond material further comprises hydrogenated rosin. In either of these embodiments, the pre-bond material optionally comprises at least one solvent selected from the group consisting of alcohols, ketones, and esters. The first embodiment preferably comprises between approximately 50 and 95 percent by weight of solvent, and between approximately 2 and 30 percent by weight of (meth)acrylate oligomer. The second embodiment preferably comprises between approximately 50 and 95 percent by weight of solvent, between approximately 2 and 30 percent by weight of (meth)acrylate oligomer, and between approximately 2 and 30 percent by weight of hydrogenated rosin.

### Radiation Curable Nail Coating Materials of the '986 Application

The following embodiments are disclosed in the'986 Application. The description also comprises inventive compositions for application to, for example, natural nails and artificial nail tips. In one embodiment, a composition comprises a polymerizable resin material, a photoinitiator, and a photoaccelerator, In another embodiment, a composition comprises: between approximately 30 and 98 percent by weight, preferably between approximately 60 to 95 percent by weight, of polymerizable resin material; between approximately 0.05 and 10 percent by weight, preferably between approximately 0.1 and 5 percent by weight, of photoinitiator; and between approximately 0.1 and 5 percent by weight, preferably between approximately 0.25 and 1 percent by weight, of photoaccelerator. In other embodiments, the polymeric material or materials comprise, for example, (meth)acrylates, and the photoinitiator comprises phosphinates, phosphine oxides and/or sulfanyl ketones (e.g., ESACURE™ 1001), and the photoaccelerator comprises aliphatic amines and/or aromatic amines, preferably ethyl 4-dimethylaminobenzoate, butoxyethyl dimethylaminobenzoate, octyl-para-dimethylaminobenzoate, and/or ethyl dimethylaminoethyl (meth)acrylate.

The compositions optionally comprise a coupling agent. Compositions comprising between approximately 0.01 and 0.5 percent by weight and preferably between approximately 0.05 and 0.15 percent by weight of a coupling agent are within the scope of the present invention. In one embodiment, the coupling agent comprises an organo-metallic, preferably an organo-titanate coupling agent such as isopropyldimethylacrylisiostearoyl titanate, tetraisopropyldi(dioctyl)phosphito titanate, neopentyl(diallyl)oxy,tri(dodecyl)benzene-sulfonyl titanate, and/or neopentyl(diallyl)oxy,trineodecanonyl titanate.

The compositions optionally comprise at least one additive such as, but not limited to, plasticizers, secondary photoinitiators, colorants, dyes, inhibitors, fillers, fibers, and/or adhesion promoting polymers. In one embodiment, the composition comprises between approximately 0 and 50 percent by weight, preferably between approximately 1 and 20 percent by weight, of additive. The compositions optionally comprise: a plasticizer such as phthalates, adipates, and/or sulfonamides; a secondary photoinitiator such as camphorquinone, benzil dimethylketal, and/or benzophenone; a colorant such as barium, calcium, aluminum lakes, iron oxides, talcs, carmine, titanium dioxide, chromium hydroxides, ferric ferrocyanide, ultramarines, titanium dioxide coated mica platelets, and/or bismuth oxychlorides; an Inhibitor such as hydroquinone, methyl ether hydroquinone, and/or butylated hydroxy toluene; a filler such as mineral fillers and/or polymeric fillers; and an adhesion promoting polymer such as methacryoyloxy ethyl phthalate.

The composition comprises a BISGMA urethane resin, a polyether, (meth)acrylated urethane resin, a photoinitiator, and a photoaccelerator. In one embodiment, the composition comprises: between approximately 30 and 90 percent by weight, preferably between approximately 50 to 70 percent by weight, of a BISGMA urethane resin; between approximately 0.5 to 50 percent by weight, preferably between approximately 10 to 40 percent by weight, of (meth)acrylated urethane resin; between approximately 0.05 to 10 percent by weight, between approximately 0.5 to 5 percent by weight, of photoinitiator; and between approximately 0.1 and 5 percent by weight, preferably between approximately 0.25 and 1 percent by weight, of photoaccelerator. In another embodiment, the composition comprises: a (meth)acrylated urethane resin having a viscosity greater than 100,000 cps; a photoinitiator such as camphorquinone, ethyl 2,4,6-trimethylbenzoyldiphenylphosphine oxide, benzildimethyl ketal, and/or benzophenone; and a photoaccelerator such as aliphatic amines and/or aromatic amines, preferably ethyl 4-dimethylaminobenzoate, butoxyethyl dimethylaminobenzoate, octyl-para-dimethylaminobenzoate, and/or ethyl dimethylaminoethyl (meth)acrylate.

Compositions optionally comprise a coupling agent, for example, but not limited to, between approximately 0.01 and 0.5 percent by weight and preferably between approximately 0.05 and 0.15 percent by weight of a coupling agent. In one embodiment, the coupling agent comprises an organo-metallic, preferably an organo-titanate coupling agent such as, but not limited to, isopropyldimethylacrylisiostearoyl titanate, tetraisopropyldi(dioctyl)phosphito titanate, neopentyl(diallyl)oxy,tri(dodecyl)benzene-sulfonyl titanate, and/or neopentyl(diallyl)oxy,trineodecanonyl titanate.

Compositions optionally comprise at least one additive such as plasticizers, secondary photoinitiators, colorants, dyes, inhibitors, fillers, fibers, and/or adhesion promoting polymers. In one embodiment, a composition comprises between approximately 0 and 50 percent by weight, preferably between approximately 1 and 20 percent by weight, of additive. In another embodiment, an inventive composition optionally comprises: a plasticizer such as phthalates, adipates, and/or sulfonamides; a secondary photoinitiator such as camphorquinone, benzil dimethylketal, and/or benzophenone; a colorant such as barium, calcium, aluminum lakes, iron oxides, talcs, carmine, titanium dioxide, chromium hydroxides, ferric ferrocyanide, ultramarines, titanium dioxide coated mica platelets, and/or bismuth oxychlorides; an Inhibitor such as hydroquinone, methyl ether hydroquinone, and/or butylated hydroxy toluene; a filler such as mineral fillers and/or polymeric fillers; and an adhesion promoting polymer such as methacryoyloxy ethyl phthalate.

### Finishing Materials of the '127 Application

The following embodiments are disclosed in the'127 Application. The description further comprises a "finishing" material for application to cured polymeric and/or urethane nail coatings. In one embodiment, the finishing material comprises at least one solvent and a plasticizer. In another embodiment the finishing material optionally comprises: between approximately 30 and 90 percent by weight, preferably between approximately 50 and 80 percent by weight, of solvent; and between approximately 5 and 40 percent by weight, preferably between approximately 10 and 30 percent by weight, of plasticizer. In yet another embodiment, the finishing material optionally comprises: a solvent such as acetone, ethyl alcohol, ethyl acetate, isopropyl alcohol, and/or methyl ethyl ketone; and a plasticizer such as phthalates, adipates, and/or sulfonamides. Further, the finishing material optionally comprises at least one fragrant material, such as, but not limited to, lavender oil.

The finishing material optionally comprises a lanolin material, or other material that softens and/or other material that protects and/or revitalizes skin. In one embodiment the inventive finishing material comprises between approximately 5 to 30 percent by weight, preferably between approximately 10 to 20 percent by weight of lanolin material. In another embodiment, the lanolin material comprises PEG-75 lanolin, hydroxylated lanolin, and/or hydrogenated lanolin. Further, the material optionally comprises at least one fragrant material, such as, but not limited to, lavender oil.

The finishing material comprises at least one solvent and a natural oil (e.g., animal and/or vegetable oil). In such an embodiment, for example, the natural oil comprises castor oil. In one embodiment the finishing material comprises: between approximately 30 and 90 percent by weight, preferably between approximately 50 and 80 percent by weight, of solvent; and between approximately 5 and 40 percent by weight, preferably between approximately 10 and 30 percent by weight, of natural oil. In yet another embodiment, the finishing material optionally comprises: a solvent such as acetone, ethyl alcohol, ethyl acetate, isopropyl alcohol, and/or methyl ethyl ketone; and a vegetable oil such as castor oil. Further, the material optionally comprises at least one fragrant material, such as, but not limited to, lavender oil.

The finishing material optionally comprises a lanolin material. In one embodiment the material comprises between approximately 5 to 30 percent by weight, preferably between approximately 10 to 20 percent by weight of lanolin material. In yet another embodiment, the lanolin material comprises PEG-75 lanolin, hydroxylated lanolin, and/or hydrogenated lanolin. Further, the material optionally comprises at least one fragrant material, such as, but not limited to, lavender oil.

The description further comprises a method to enhance polished characteristics of a coated natural nail or nail tip. In one embodiment, the method comprises the steps of: applying a material comprising at least one solvent and a plasticizer to the coated natural nail or nail tip; and cleaning the coated natural nail or nail tip, preferably with a clean cloth or cotton. The applied material comprises between approximately 30 and 90 percent by weight of solvent, such as acetone, ethyl alcohol, ethyl acetate, isopropyl alcohol, and methyl ethyl ketone, and between approximately 5 and 40 percent by weight of plasticizer, such as phthalates, adipates, and sulfonamides. The applied material optionally comprises a fragrant material, such as, but not limited to, lavender oil.

The method comprises the steps of: applying a material comprising at least one solvent, a plasticizer, and a lanolin material to the coated natural nail or nail tip; and cleaning the coated natural nail or nail tip, preferably with a clean cloth or cotton. The applied material optionally comprises between approximately 30 and 90 percent by weight of solvent, such as acetone, ethyl alcohol, ethyl acetate, isopropyl alcohol, and methyl ethyl ketone, between approximately 5 and 40 percent by weight of plasticizer, such as phthalates, adipates, and sulfonamides, and between approximately 5 and 30 percent by weight of a lanolin material, such as PEG-75 lanolin, hydroxylated lanolin and/or hydrogenated lanolin. The appiled material optionally comprises a fragrant material, such as, but not limited to, lavender oil.

The method comprises the steps of: applying a material comprising at least one solvent and a vegetable oil to the coated natural nail or nail tip; and cleaning the coated natural nail or nail tip, preferably with a clean cloth or cotton. The appiled material optionally comprises between approximately 30 and 90 percent by weight of solvent, such as acetone, ethyl alcohol, ethyl acetate, isopropyl alcohol, and methyl ethyl ketone, and between approximately 5 and 40 percent by weight of vegetable oil, such as castor oil. The applied material optionally comprises a fragrant material, such as, but not limited to, lavender oil.

The method comprises the steps of: applying a material comprising at least one solvent, a vegetable oil, and a lanolin material to the coated natural nail or nail tip; and cleaning the coated natural nail or nail tip, preferably with a clean cloth or cotton. The applied material optionally comprises between approximately 30 and 90 percent by weight of solvent, such as acetone, ethyl alcohol, ethyl acetate, isopropyl alcohol, and methyl ethyl ketone, between approximately 5 and 40 percent by weight of vegetable oil, such as castor oil, and between approximately 5 and 30 percent by weight of a lanolin material, such as PEG-75 lanolin, hydroxylated lanolin and/or hydrogenated lanolin. The applied material optionally comprises a fragrant material, such as, but not limited to, lavender oil.

### Radiation Curable Compositions

The description relates to actinic (e.g., visible and UV) radiation curable coating materials used to coat artificial nail tips and extend, strengthen and coat natural nails. Of course, electron beam (EB) curable materials are also within the scope of the present invention. The materials are optionally applied to the natural nail or to the natural nail and a pre-formed nail tip that is attached to the natural nail. The coatings react with actinic radiation, even when highly colored. The coating materials can be formulated in clear, opaque white, translucent colors and opaque colors, and cure, in some cases, in less than approximately two minutes with UV radiation. The coating materials are polymeric and/or urethane based. The term "polymeric materials" as used throughout the specification and claims, is Intended to include resins, monomers, oligomers, and polymers. For example, a resin is a polymeric material, natural and/or synthetic. The term "resin" as used throughout the specification and claims, also generally includes "oligomers," which are molecules having a relatively intermediate molecular mass.

A biocompatible solvent-based adhesive resin (pre-bond material) is used to enhance the bond between the radiation curable nail coatings and the natural nail. The natural nails are preferably prepared by filing, and a thin coat of the solvent-based adhesive resin is applied to the surface of the natural nail beginning at the cuticle area. The solvent evaporates leaving a sticky layer of the adhesive. The radiation curable nail coating is then applied to the adhesive. In another embodiment incorporating artificial nail tips, the artificial nail tip is attached to the natural nail as is known in the art. A thin coat of the solvent-based adhesive resin is applied to the surface of the natural nail beginning at the cuticle area and ending at the front edge of the nail tip. There is no need to apply the adhesive resin to the surface of the nail tip. The solvent evaporates leaving a sticky layer of the adhesive. The radiation curable nail coating is then applied to the adhesive on the surface of the natural nail as well as the nail tip. The use of the adhesive resin enhancer prevents lifting of the cured coating from the natural nail, adding longevity and beauty.

The pre-bond materials can also be used to enhance the adhesive properties of polymeric filler materials used to treat animal hoofs and claws. In one application, the pre-bond material is initially added to a crack or hole in the hoof, e.g., a horse hoof, and the solvent is allowed to partially evaporate resulting in a tacky surface. The polymeric filler materials are then added to fill the voids in the hoof or claw. The hoof or claw can then be filed to the desired shape and appearance.

### Pre-Bond Materials

The description comprises pre-bond materials that are applied to the natural nail to enhance the adhesive properties of the coating materials. The pre-bond materials comprise a (meth)acrylate based polymer, preferably an aromatic acid (meth)acrylate, in a biocompatible solvent. The preferred aromatic acid (meth)acrylates are commercially available as SARBOX® SB 500E50 or SARBOX® 600 from Sartomer Company located in Exton, PA. SARBOX® SB 500E50 is a proprietary aromatic acid (meth)acrylate half ester in ethoxylated trimethylolpropane triacrylate monomer (see above). SARBOX® 600 is a proprietary aromatic acid (meth)acrylate half ester in a trifunctional (meth)acrylate monomer. The acid (meth)acrylate-triacrylate monomer mixture or the acid (meth)acrylate- trifunctional (meth)acrylate mixture is present in the material between approximately 2 and approximately 30% by weight and preferably between approximately 5 and approximately 20% by weight. In another embodiment hydrogenated rosin is added to the solvent and (meth)acrylate oligomer mixture. The preferred hydrogenated rosin is commercially available in the form of a glycerol ester from Hercules, formulated as FORAL® 85 synthetic resin, and is ground to a powder prior to the addition. The hydrogenated rosin, if added, is present In the material between approximately 2 and approximately 30% by weight and preferably 5 and approximately 20% by weight. The solvent in the material is biocompatible and evaporates rapidly after being applied to the natural nail. It may be a single organic solvent or blend of organic solvents. The solvent is preferably selected from the group consisting of alcohols, ketones and esters. In one embodiment, the biocompatible solvent is acetone and comprises between approximately 50 and approximately 95% by weight and preferably between approximately 60 and approximately 80% by weight, of the material.

The pre-bond materials are used in conjunction with the coatings of the present invention to greatly enhance the bonding of the coatings to the natural nail. As a result, lifting of the coating at the cuticle area is significantly reduced. The following pre-bond materials, useful in accordance with the present invention, are shown in Table 1 in varying percentages. The material of Example 1 describes a pre-bond material that Improves adhesion of the radiation cured coating to natural nails. The material of Examples 2 and 2A have more surface tackiness after application and evaporation. The material of Example 2A is all (meth)acrylate version which is less likely to cause skin sensitization. The materials of Examples 2 and 2A give greatly improved adhesion of the radiation cured coating to the natural nail with little or no lifting during normal wear.

**Table 1. Pre-Bonding Examples 1, 2, 2A (quantities in grams)**

| Ingredient | 1 | 2 | 2A |
|---|---|---|---|
| Acetone | 8.52 | 52.43 | 14.0 |
| SARBOX® 500E50 | 1.52 | 11.18 | -- |
| SARBOX® 600 | -- | -- | 3.0 |
| Hydrogenated Rosin | -- | 11.33 | 3.0 |

### Coating Materials

The description relates to actinic radiation, preferably UV radiation, curable materials used to coat natural nails and pre-formed artificial nail tips. The nail tips are attached to the natural nail with a known cyanoacrylate gel material (e.g. see, U.S. Patent No. 4,704,303, entitled "Nail extension composition," to Cornell, issued November 3,1987. The coatings of the present invention are durable and do not result in skin sensitivity in the majority of the population. In the preferred embodiment, the coating materials comprise: (a) polymerizable (meth)acrylate resins; (b) a photoinitiator, preferably a phosphinate, phosphine oxide, and/or sulfanyl ketone (ESCURE™ 1001) photoinitiator; and (c) a photoaccelerator, preferably an aliphatic or aromatic amine photoaccelerator such as ethyl 4-dimethylaminobenzoate, butoxyethyl dimethylaminobenzoate, octyl-para-dimethylaminobenzoate, and ethyl dimethylaminoethyl (meth)acrylate. The coating materials may further comprise a coupling agent, preferably, a titanate coupling agent, and various other additives such as plasticizers, other photoinitiators, colorants, dyes, inhibitors, fillers, fibers, rosins, solvents, and adhesion promoting monomers.

The material preferably comprises: 1) between approximately 30 and approximately 99% by weight and preferably between approximately 60 and approximately 95% by weight of component (A); 2) between approximately 0.05 and approximately 10% by weight and preferably between approximately 0.1 and approximately 5% by weight of component (B); and 3) between approximately 0.1 and approximately 5% by weight and preferably between approximately 0.25 and approximately 1% by weight of component (C). The one embodiment may further comprise between approximately 0.01 and approximately 0.5% by weight and preferably between approximately 0.05 and approximately 0.15% by weight of a coupling agent (component (D)); and between approximately 0 and approximately 50% by weight and preferably between approximately 1 and approximately 20% by weight of other additives (component (E)).

Component (A) is preferably a polymerizable (meth)acrylate resin. Component (B) is preferably an acyl phosphine oxide such as 2,4,6-trimethyl benzoyldiphenylphosphine oxide or an alkyl phosphinate such as ethyl 2,4,6-trimethyl benzoylphenylphosphinate, commonly available from BASF. Component B may also be a sulfanyl ketone such as 1-[4-(4-benzoylphenylsulfanyl) phenyl]-2-methyl-2-(4-methylphenylsulfanyl) propan-1-one available as ESACURE™ 1001 from Lamberti spa in Italy. Component (C) Is preferably an aliphatic or aromatic amine photoaccelerator such as ethyl 4-dimethylamino benzoate, butoxyethyl dimethylaminobenzoate, octyl-para-dimethylaminobenzoate or ethyl dimethyl aminoethyl (meth)acrylate. The preferred coupling agent, component (D), is an organic titanate coupling agent such as a class of neoalkoxy titanates available from Kenrich Petrochemicals. Additional and optional additives, component (E), may include: plasticizers such as the phthalates, adipates and sulfonamides; other photoinitiators such as camphorquinone and benzil dimethylketal, benzophenone; inhibitors such as hydroquinone, methyl ether hydroquinone and butylated hydroxy toluene; mineral and polymeric fillers; fibers; rosins such as glycerol esters of hydrogenated rosins, solvents such as ethyl acetate, acetone and isopropyl alcohol, adhesion promoting monomers such as methacryoyloxy ethyl phthalate; and colorants such as barium, calcium and aluminum lakes, iron oxides, talcs, carmine, titanium dioxide, chromium hydroxides, ferric ferrocyanide, ultramarines, titanium dioxide coated mica platelets and bismuth oxychlorides.

The coating material is based on the combination of a polymerizable BISGMA urethane and an aliphatic (meth)acrylated urethane (optionally polyol modified, e.g., polyether, polyester, and/or polycarbonate) with a preferred viscosity greater than 80,000 cps. The BISGMA urethane based nail coating, even a coating that is heavily pigmented, reacts with UV radiation. A coat of the material cures with a standard ultraviolet nail light in less than two minutes. Moreover, it cures with visible light, such as from a dental curing unit, in less than 20 seconds.

A material is described that comprises: 1) a resin, preferably a polymerizable BISGMA urethane resin, prepared from an adduct of BISGMA and an aliphatic or cycloaliphatic hydrocarbon diisocyanate (component (A)); 2) a polyether (or other polyol) based, (meth)acrylated aliphatic urethane resin with a viscosity greater than 80,000 cps (component (B)); 3) a photoinitiator (component (C)); and 4) a photoaccelerator (component (D)). This embodiment may further comprise a coupling agent (component (E)) and various other additives such as plasticizers, colorants, dyes, inhibitors, fillers, fibers, rosins, solvents, adhesion promoting monomers and crosslinking monomers (components (F)). The coating preferably comprises between approximately 30 and approximately 90% by weight of and preferably between approximately 50 and approximately 70% by weight of component (A); between approximately 0.5 and approximately 50% by weight and preferably between approximately 10 and approximately 40% by weight of component (B); between approximately 0.05 and approximately 10% by weight and preferably between approximately 0.5 and approximately 5% by weight of component (C); and between approximately 0.1 and approximately 5% by weight and preferably between approximately 0.25 and approximately 1 % by weight of component (D). The coating may further comprise a coupling agent (component (E)) in an amount of between approximately 0.01 and approximately 0.5% by weight and preferably between approximately 0.05 and approximately 0.15% by weight. Further, the material may optionally comprise between approximately 1 and approximately 50% and preferably between approximately 5 and approximately 20% by weight of any one or combination of components (F).

A material is described that comprises: 1) a resin, preferably a BISGMA urethane resin, prepared from an adduct of BISGMA and an aliphatic or cycloaliphatic hydrocarbon diisocyanate (component (A)); 2) polyether (or other polyol) based, (meth)acrylated aliphatic urethane resins of varying molecular weights with viscosities greater than 80,000 cps (component (B)); 3) a photoinitiator (component (C)); and 4) a photoaccelerator (component (D)). This embodiment may further comprise a coupling agent (component (E)) and various other additives such as plasticizers, colorants, dyes, inhibitors, fillers, fibers, adhesion promoting monomers and crosslinking monomers (components (F)). The coating preferably comprises between approximately 10 and approximately 60% by weight of and preferably between approximately 20 and approximately 50% by weight of component (A); between approximately 30 and approximately 70% by weight and preferably between approximately 40 and approximately 60% by weight of component (B); between approximately 0.05 and approximately 10% by weight and preferably between approximately 0.5 and approximately 5% by weight of component (C); and between approximately 0.1 and approximately 5% by weight and preferably between approximately 0.25 and approximately 1% by weight of component (D). The coating may further comprise a coupling agent (component (E)) in an amount of between approximately 0.01 and approximately 0.5% by weight and preferably between approximately 0.05 and approximately 0.15% by weight. Further, the material may optionally comprise between approximately 1 and approximately 50% and preferably between approximately 5 and approximately 25% by weight of any one or combination of components (F).

A material is described that comprises: 1) a resin, preferably a BISGMA urethane resin, prepared from an adduct of BISGMA and an aliphatic or cycloaliphatic hydrocarbon diisocyanate (component (A)); 2) polyether based (or other polyol), (meth)acrylated aliphatic urethane resins of varying molecular weights with viscosities greater than 80,000 cps (component (B)); 3) a photoinitiator (component (C)); and 4) a photoaccelerator (component (D)). This embodiment may further comprise a coupling agent (component (E)) and various other additives such as plasticizers, colorants, dyes, inhibitors, fillers, fibers, rosins, solvents, adhesion promoting monomers and crosslinking monomers (components (F)). The coating preferably comprises between approximately 0 and approximately 40% by weight of and preferably between approximately 5 and approximately 30% by weight of component (A); between approximately 20 and approximately 80% by weight and preferably between approximately 30 and approximately 70% by weight of component (B); between approximately 0.05 and approximately 10% by weight and preferably between approximately 0.5 and approximately 5% by weight of component (C); and between approximately 0.1 and approximately 5% by weight and preferably between approximately 0.25 and approximately 1% by weight of component (D). The coating may further comprise a coupling agent (component (E)) in an amount of between approximately 0.01 and approximately 0.5% by weight and preferably between approximately 0.05 and approximately 0.15% by weight. Further, the material may optionally comprise between approximately 1 and approximately 50% and preferably between approximately 5 and approximately 30% by weight of any one or combination of components (F).

BISGMA based urethane resins are disclosed in U.S. Patent No. 3,629,187, entitled "Dental compositions containing adduct of 2,2' - propane bis 3-(4-phenoxy)-1,2-hydroxy proane-1-(meth)acrylate and isocyanate," to Weller, issued December 21, 1971 and U.S. Patent No. 3,709,866, entitled "Polymerizable dental products," to Waller, issued January 9, 1973.

BISGMA based urethane resin is preferably prepared by reacting the hydroxyl functions of BISGMA with a hydrocarbon diisocyanate. In one embodiment, the BISGMA is diluted with di(meth)acrylate monomers, a catalyst is added and then the diisocyanate compound is slowly added. The reaction mixture is heated (approximately 55 °C) until all the diisocyanate has reacted to the BISGMA urethane. BISGMA can be purchased from Esstech, and is also sold as Nupol 46-4005 from Cook Composites and Polymers. BISGMA is a very thick, sticky, liquid which must be diluted with di(meth)acrylate monomers prior to the addition of the diisocynate. The di(meth)acrylate monomers are well suited for the dilution step because they exhibit relatively low volatility and low odor. The di(meth)acrylate monomers also serve as crosslinking agents in the urethane resin. Suitable di(meth)acrylate monomers are diethylene glycol di(meth)acrylate, 1,6-hexane diol di(meth)acrylate, triethylene glycol di(meth)acrylate, ethoxylated bisphenol A di(meth)acrylate, polyethylene glycol di(meth)acrylate, and 1,4-butanediol di(meth)acrylate. The final urethane resin comprises between approximately 30 and approximately 70% di(meth)acrylate monomers based on the weight of the BISGMA adduct in the urethane resin.

Urethane catalysts are tin compounds such as dibutyl tin dilaureate and stannous octoate. They are used at levels between approximately 0.005 and approximately 0.10% by weight in the urethane resin. The tin compound is added to the mixture of BISGMA and di(meth)acrylate monomers and mixed. The diisocyanate is an aliphatic or cycloaliphatic hydrocarbon such as heptyl diisocyanate, trimethylhexamethylene diisocyanate, or isophorone diisocyanate. The diisocyanate is slowly added to the BISGMA, di(meth)acrylate monomer and catalyst mixture to form the urethane. The diisocyanate is used at levels of between approximately 5 and approximately 12% by weight of the urethane resin. Alternatively, the diisocyanate may be diluted with di(meth)acrylate monomer to control the exothermic urethane reaction. Once the urethane reaction is completed, a small amount of inhibitor, preferably butylated hydroxy toluene, in an amount of between approximately 0.01 and approximately 0.10% by weight, is added.

The polyether (or other polyol) based (meth)acrylated aliphatic urethane resin(s) with a viscosity greater than 80,000 cps are then mixed with the BISGMA based urethane. The photoinitiator is then added, preferably camphorquinone, ethyl 2,4,6-trimethylbenzoylphenylphosphinate, 2,4,6-trimethylbenzoyl diphenylphosphine oxide, 1-[4-(4-benzoylphenylsulfanyl)phenyl]-2-methyl-2-(4-methylphenylsulphonyl) propan-1-one (e.g., ESACURE 1001, Lamberti spa, Italy), benzil dimethyl ketal and/or benzophenone. The remaining ingredients are also added: plasticizers such as phthalates, adipates, and sulfonamides; aliphatic or aromatic amine photoaccelerators such as ethyl 4-dimethylaminobenzoate, butoxyethyl dimethylaminobenzoate, octyl-para-dimethyleminobenzoate, and ethyl dimethylaminoethyl (meth)acrylate; titanate coupling agents such as isopropyl dimethacryl isostearoyl titanate, tetraisopropyl di(dioctyl)phosphito titanate, neopentyl(diallyl)oxy-tri(dodecyl)benzene-sulfonyl titanate, and neopentyl(diallyl)oxy-trineodecanonyl titanate; inhibitors such as hydroquinone, methyl ether hydroquinone and butylated hydroxy toluene; mineral and polymeric fillers; fibers; rosins such as glycerol esters of hydrogenated rosins, solvents such as ethyl acetate, acetone and isopropyl alcohol, adhesion promoting monomers such as methacryoyloxy ethyl phthalate; colorants such as barium, calcium, aluminum lakes, iron oxides, talcs, carmine, titanium dioxide, chromium hydroxides, ferric ferrocyanide, ultramarines, titanium dioxide coated mica platelets, and bismuth oxychlorides.

### Finishing Materials

The description recites nail finishing materials that complement the actinic radiation curable coatings. After the coating has been applied and cured with actinic radiation, oxygen inhibition causes the surface of the coating to become tacky. Typically, isopropyl alcohol is used to remove the sticky layer. As an alternative, the compositions of the finishing materials may be applied with a cotton swab or pad, or sprayed directly onto the nail to remove the sticky surface. The nail is then rubbed or buffed, for example, with a clean cloth, resulting In a clean smooth surface. A high gloss surface results using the finishing materials of the present invention as compared to using 100% isopropyl alcohol. The nail treatment may be considered complete at this point. If higher gloss is desired, a topcoat of a solvent-based cellulose material may be applied to complete the procedure.

The finishing materials are applied to the cured coating to provide a sleek, high gloss surface, Table 2. The finishing materials preferably comprise: 1) between approximately 30 and approximately 90% by weight and preferably between approximately 50 and approximately 80% by weight, of a biocompatible solvent such as acetone, ethyl alcohol, ethyl acetate, isopropyl alcohol, and methyl ethyl ketone; 2) between approximately 5 and approximately 40% by weight and preferably between approximately 10 and approximately 30% by weight, of a plasticizer such as phthalates, adipates and sulfonamides, or an oil, such as castor oil; and 3) between approximately 5 and approximately 30% by weight and preferably between approximately 10 and approximately 20% by weight, of a lanolin based material such as PEG-75 lanolin, hydroxylated lanolin, hydrogenated lanolin, and other lanolin derivatives. A fragrance, such as lavender oil, may also be added to make this material more appealing to the consumer. Some representative formulas for the finishing materials are shown in Table 2.

The finishing material of Example 3 comprises approximate equal proportions by weight of isopropyl alcohol and the plasticizer, dioctyl adipate. The finishing material of Example 4 comprises approximate equal proportions by weight of ethyl alcohol and the plasticizer, dioctyl adipate with smaller amounts of a lanolin based material. The finishing material of Example 5 is similar to that of Example 4 with the exception that isopropyl alcohol is substituted for the ethyl alcohol. The finishing material of Example 6 substitutes castor oil for the plasticizer in the prior compositions and also includes a fragrant material.

**Table 2. Examples 3 through 6 (quantities in grams)**

| Example | 3 | 4 | 5 | 6 |
|---|---|---|---|---|
| Ethyl Alcohol | -- | 8.56 | - | -- |
| Isopropyl alcohol | 3.82 | -- | 3.73 | 49.83 |
| Lanolin PEG-75 | -- | 1.25 | 0.55 | 8.97 |
| Dioctyl Adipate | 3.92 | 11.50 | 4.97 | -- |
| Castor Oil | -- | -- | -- | 8.66 |
| Lavender Oil | -- | -- | -- | 0.62 |

### Method of Creating a Permanent Nail Enhancement

Materials are applied to a natural nail, wherein the nail is optionally and preferably filed on the top surface to remove oils and create a surface for bonding. A bond-enhancing material is applied to the entire surface of the nail, preferably beginning at the cuticle. The solvent in the pre-bond finishing material is allowed to evaporate over a given period, leaving a tacky surface. An optional actinic radiation curable white tipping coating is then applied to the ends of the nail and cured if the French manicure look is desired. A clear or light pink coating material is applied to the nail, preferably starting at the cuticle and moving toward the white tipping, and cured. The coated nail is then filed to a smooth surface. A clear coating material is applied over the entire nail surface and cured. Application of a second clear coat is optional. The finishing material is applied to remove the top sticky layer and the coated nail is buffed to a high gloss shine.

Materials are applied to a nail-tip that is attached to natural nail, wherein the natural nail is optionally filed on the top surface to remove oils and create a surface for bonding. The nail-tip Is applied to the natural nail with, for example, but not limited to, a glue (e.g. cyanoacrylate glue) known in the art. After the glue has set, the nail-tip is optionally filed. A bond-enhancing material is optionally applied to the natural nail, preferably beginning at the cuticle to the front edge of the nail-tip. It is not necessary to apply the bond-enhancing material to the surface of the nail tip. An optional actinic radiation curable white tipping coating is applied to the ends of the nail-tip and cured. A clear or light pink coating material is applied to the entire nail, preferably starting at the cuticle and moving toward the white tipping, and cured. The coated nail is then filed to a smooth surface. A clear coating material is applied over the entire nail surface and cured. Application of a second clear coat is optional. The finishing material is applied to remove the top sticky layer and the coated nail is buffed to a high gloss shine.

Methods of applying colored coating materials of the present invention are similar to the methods described above for the clear-white tipped nails except, for example, that a second final clear coat optionally replaces a colored coating. Application of an optional second colored coat is applied for best results.

**Table 3. Examples 7 through 15 (quantities in grams)**

| Ingredient | 7 Prior Art | 8 Prior Art | 9 not claimed | 10 | 11 | 12 | 13 | 14 | 15 |
|---|---|---|---|---|---|---|---|---|---|
| UDMA resin | 3.01 | 3.79 | 12.56 | | -- | -- | -- | -- | -- |
| Ethoxylated BPADMA | 4.04 | 4.61 | 6.00 | -- | -- | -- | -- | -- | -- |
| Benzildimethyl ketal | .0316 | -- | .04 | .0771 | .0211 | .1920 | .0470 | .0392 | .0283 |
| Butoxyethyldimethylaminobenzoate | .09 | -- | -- | -- | -- | -- | -- | -- | -- |
| Dimethylaminoethyl(meth)acrylate | -- | .13 | -- | -- | -- | .51 | .13 | .1074 | .0776 |
| Octyl-para-dimethyl aminobenzoa te | -- | - | .14 | .2264 | .0649 | -- | -- | -- | -- |
| Ethyldimethylaminobenzoate | -- | -- | -- | -- | -- | -- | -- | .0311 | |
| Ethyl 2,4,6-trimethylbenzoylphenylphosphinate | -- | .09 | .17 | .2459 | .0674 | .52 | .13 | .11 | .08 |
| Neoalkoxytitanate | -- | -- | .02 | .0300 | .0084 | .11 | .0300 | .0248 | .0179 |
| D & C Red #33 | -- | -- | -- | -- | .0136 | -- | -- | -- | |
| D & C Red #6 Barium Lake | -- | -- | -- | -- | -- | -- | -- | .0495 | -- |
| D & C Red #7 Calcium Lake | -- | -- | - | -- | -- | -- | -- | .1127 | -- |
| Ext. D & C Violet #2 | -- | -- | -- | -- | -- | -- | -- | .0015 | -- |
| Titanium dioxide coated mica platelets | -- | -- | -- | -- | -- | -- | -- | -- | .0750 |
| Titanium dioxide | -- | -- | .07 | -- | -- | -- | .0374 | .0300 | .0075 |
| N-ethyl-o & p toluenesulfonamide | -- | -- | 1.00 | 2.10 | 0.59 | 7.09 | 1.75 | 1.46 | 1.05 |
| Mono(Methacryl-oyloxyethyl)Phthalate | -- | -- | -- | -- | -- | 2.67 | .66 | .55 | .40 |
| (meth)acrylated-aliphatic Urethane Resin | -- | -- | -- | 4.50 | 2.52 | 30.43 | 7.46 | 6.22 | 4.50 |
| BISGMA Urethane Resin of the Invention | -- | -- | -- | 22.83 | 5.15 | 59.8 | 14.54 | 12.13 | 8.77 |

The formulas shown in Examples 7 and 8 illustrate typical UV radiation curable resin systems, known In the art. The materials of Examples 7 and 8 cure under UV radiation in about two to three minutes. The formulas of Examples 10 to 15 illustrate coating materials used in the present invention. The material of Example 9 is highly reactive to UV radiation and will cure in less than two minutes, even with a high level of titanium dioxide white pigment. The formulas of Examples 10 through 12 are for coating materials with the BISGMA urethane resin and the polyether based (meth)acrylated aliphatic urethane resin. The latter resin having a viscosity greater than 80,000 cps. These materials are easily applied and exhibit a creamy consistency, which do not run on the nail during application, unlike the prior art materials of Examples 7 and 8. The materials of Examples 10 through 12 exhibit clear and translucent pink shades that are easily cured with UV radiation in less than two minutes to a durable nail coating. The formula of Example 13 is for a UV radiation cured opaque white material useful for covering the very end of the natural nail or nail tip to give the French manicure look. The formula of Example 14 is for a very highly colored material and the formula of Example 15 is for a shimmering or opalescent material, both of which cure to form durable coatings when exposed to UV radiation for approximately two minutes.

### Description of Examples 16 through 21

Table 4 lists formulas for Examples 16 through 21. The formulas of Examples 16, 17, and 18 comprise a substantially clear material for application to a nail. The formulas of Examples 16, 17, and 18 harden in approximately 1 to approximately 2 minutes under ultraviolet radiation. The formulas of Examples 19, 20, and 21 comprise a substantially opaque white material for application to a nail. The formulas of Examples 19, 20, and 21 harden in approximately 2 to approximately 3 minutes under ultraviolet radiation. The formulas of Examples 16-21 are further optionally useful for creating a free edge of a nail. In general, the formulas of Examples 18 and 19 are less brittle than the formulas presented in the '986 Application. Overall, the materials of Examples 16 through 21 are optionally useful in creating a high gloss natural look and giving strength to the nails.

**Table 4. Examples 16 through 21 (quantities in grams)**

| Ingredient | 16 | 17 | 18 | 19 | 20 | 20A | 20B | 21 |
|---|---|---|---|---|---|---|---|---|
| BISGMA Urethane resin | 22.83 | 59.8 | 2.56 | 7.08 | 14.54 | 88.65 | 88.85 | 6.25 |
| (meth)acrylated polyether urethane Resin | 4.5 | 30.43 | 3.57 | 9.80 | 7.46 | 44.0 | 44.13 | 3.58 |
| Ethyl toluenesulfonamide | 2.10 | 7.09 | 0.41 | 1.12 | 1.75 | 10.7 | 10.7 | 0.84 |
| Octyl-para-dimethylamino Benzoate | 0.2264 | -- | -- | -- | -- | -- | -- | -- |
| Benzildimethylketal | 0.0771 | 0.1920 | 0.0131 | 0.0358 | 0.0470 | 0.28 | 0.28 | 0.0227 |
| Ethyl 2,4,6-trimethylbenzoyl-Phenylphosphinate | 0.2459 | 0.52 | 0.0346 | 0.1160 | 0.13 | 0.74 | 0.74 | 0.062 |
| Neoalkoxy titanate | 0.0300 | 0.11 | 0.0092 | 0.0209 | 0.0300 | 0.19 | 0.19 | 0.12 |
| Fumed silica | -- | -- | -- | -- | -- | -- | -- | 0.42 |
| Hydrogenated Rosin | -- | -- | 0.0281 | 0.0768 | -- | -- | -- | -- |
| Ethyl Acetate | -- | -- | 0.0138 | 0.0378 | -- | -- | -- | -- |
| Mono(methacryloyl-Oxyethyl)phthalate | -- | 2.67 | 0.1932 | 0.5143 | 0.66 | 4.03 | 4.03 | 0.33 |
| Dimethylaminoethyl(meth)acrylate | -- | 0.51 | 0.0345 | 0.0967 | 0.13 | 0.74 | 0.74 | 0.0621 |
| Iron Oxide and mica | -- | -- | -- | -- | -- | 0.30 | 0.12 | -- |
| D & C Red #7 Calcium Lake | -- | -- | -- | --- | -- | 0.15 | -- | -- |
| Titanium dioxide | -- | -- | -- | 0.00947 | 0.0374 | 0.30 | 0.30 | 0.0604 |

Examples 22 through 26 list formulas that can be applied to natural or artificial nails prior to the application of radiation cured nail coatings to aid bonding.

**Table 5. Examples 22 through 26 (quantities In grams)**

| Ingredient | 22 | 23 | 24 | 25 | 26 |
|---|---|---|---|---|---|
| Acetone | 8.52 | 52.43 | 8.18 | 11.81 | 2.38 |
| Sarbox 500E50 | 1.52 | 11.18 | 1.75 | 2.53 | 0.51 |
| Hydrogenated Rosin | -- | 11.33 | 1.78 | 2.53 | 0.51 |
| BISGMA Urethane Resin | -- | -- | 0.46 | 0.33 | -- |
| (meth)acrylated polyether urethane resins | -- | -- | 0.35 | 1.67 | -- |
| Ethyl 2,4,6-trimethylbenzoyl-Phenylphosphinate | -- | **--** | 2.38 | 0.0111 | -- |
| Dimethylaminoethyl (meth)acrylate | -- | -- | 0.0153 | 0.0112 | -- |
| Ethyl toluenesulfonamide | -- | -- | 0.15 | 0.1561 | -- |
| Neolkoxy titanate | -- | -- | 0.0032 | 0.0022 | -- |
| Mono(methacryloyl-oxyethyl) phthalate | -- | -- | 0.0919 | 0.0691 | -- |
| Ethyl Acetate | -- | -- | 0.0131 | -- | -- |
| Tosylamide Epoxy Resin | -- | -- | -- | 4.66 | 0.98 |

### Method Of Creating a Permanent Nail Enhancement with Color

The following method describes the use of Examples 16-18, 20A and 20B to create a permanent nail enhancement with color that can be filed off, but not soaked off:
Step 1: File natural nail to remove oil and shine to minimize any subsequent lifting.
Step 2: If desired, select a nail tip and/or nail form:
   Step 2a: Apply the nail tip or form using, for example, an adhesive (glue)
   Step 2b: If using a nail tip, cut the nail tip to a desired shape
   Step 2c: Blend (e.g., file) the nail tip to natural nail
Step 3: Select a bond agent, for example, from one of the formulas presented in Examples 1-2A or Examples 22-26.
Step 4: Apply the bond agent to natural nail, which comprises a nail tip and/or nail form. Do not cure.
Step 5: Select clear material, for example, from one of the formulas presented in Examples 16 through 18.
Step 6: Apply the selected clear material in one to two coats curing clear material between coats for at least approximately 1 to approximately 2 minutes.
Step 7: Remove residue from nail with, for example, alcohol.
Step 8: If a nail form was applied then:
   Step 8a: Remove the nail form.
Step 9: Shape and buff cured material applied to the nail into a desired shape.
Step 10: Select a formula from formulas presented in Examples 16, 17, 20A or 20B.
Step 11: Apply the selected formula from Step 10 on the already treated nails; preferably apply to one to two fingers at a time; preferably, use a brush and only one side of brush to allow for a closer approach to cuticle.
Step 12: Cured the applied formula from Step 11.
Step 13: Optionally repeat Steps 11 and 12.
Step 14: Remove any residue from the coated nails with, for example, alcohol or finishing compounds of Examples 3-6.
Step 15: Apply a solvent evaporation based high sheen coatings for nails for greater shine if desired.

### Method of Creating a Permanent Nail Enhancement with a French White Look

The following method describes the use of Examples 16, 17, 18, 19, 20, and 21 to create a permanent nail enhancement with a French White Look that can be filed off, but not soaked off:
Step 1: File natural nail to remove oil and shine to minimize any subsequent lifting.
Step 2: If desired, select a nail tip and/or form:
   Step 2a: Apply the nail tip or nail form;
   Step 2b: If using a nail tip, then cut the nail tip to a desired shape; and
   Step 2c: Blend the nail tip to natural nail.
Step 3: Select a white tipping agent from, for example, the formulas presented in Example 19, 20, or 21.
Step 4: Apply white tipping to the nail form or nail tip, creating a smile line at the free edge.
Step 5: Cure the applied white tipping agent for approximately 2 minutes.
Step 6: Optionally repeat Step 4 and 5 to form a more definite smile line at free edge.
Step 7: Select a bond agent, for example, from one the formulas presented in Examples 1-2A, or 22 through 26.
Step 8: Apply the selected bond agent to natural nail comprising the applied nail form and/or nail tip. Do not cure.
Step 9: Select clear material, for example, from one of the formulas presented In Examples 16 through 18.
Step 10: Apply the selected clear material In approximately one to approximately two coats, curing between coats for at least approximately one to approximately 2 minutes.
Step 11: Remove any residue from the treated nail with, for example, alcohol.
Step 12: If nail form was applied then:
   Step 12a: Remove the nail form.
Step 13: Cure the applied material;
Step 14: Shape and buff cured material applied to the nail into a desired shape.
Step 15: Select a final coating material from one of the formulas in Examples 16, 17, 18, 20A or 20B.
Step 16: Apply the selected final coating material from Step 15 onto the treated nails (per Steps 1 through 13) applying the coated material, for example, to one to three fingers at a time.
Step 17: Cure the applied coating material for approximately 1 to approximately 2 minutes.
Step 18: Repeat Step 16 and 17 until all nails have been coated.
Step 19: Remove residue from the coated nail with, for example, alcohol or finishing compounds from Examples 3-6.
Step 20: Apply a solvent evaporation based high sheen coatings for nails if greater shine is desired.

### Method Of Creating a Permanent Nail Enhancement with a French White Look Using an Electric File

The following method describes the use of Examples 16, 17, 18, 19, 20, and 21 to create a permanent nail enhancement with the French White Look using the electric file that can be removed by
filing, but not soaked off:
Step 1: File natural nail to remove oil and shine to minimize any subsequent lifting.
Step 2: If desired, select a nail tip and/or nail form:
   Step 2a. Apply the nail tip or form
   Step 2b. If using a nail tip, cut the nail tip to desired length; and
   Step 2c. Blend and shape nail tip to natural nail
Step 3: Select a bond agent, for example, from one of the formulas presented in Examples 1-2A or 22-26.
Step 4: Apply the bond agent to natural nail, which comprises a nail tip and /or nail form. Do not cure.
Step 5: Select clear material, for example, from one of the formulas presented in Example 16-18.
Step 6: Apply the selected clear material in one or two coats, curing the clear material between coats for at least approximately 1 to approximately 2 minutes.
Step 7: Remove any residue from the nail with, for example, alcohol.
Step 8: If a nail form was applied then:
   Step 8a. Remove nail form
Step 9: Shape and buff cured material applied to nail into a desired shape.
Step 10: Creating a French look use an electric file, removing clear material previously applied in Step 6 at free edge only, to create a line and/or ditch.
Step 11: Select a formula from Example 19, 20 or 21 to fill in area previous filed out with an electric file.
Step 12: With selected formula, apply material at area where product was removed to create a white French tip.
Step 13: Cure formula applied In Step12, for approximately for 2 to approximately 3 minutes.
Step 14: Remove any residue from the coated nails with, for example, alcohol.
Step 15: File and buff nail lightly to smooth out any unevenness of nail.
Step 16: Select a final coating material from one of the formulas In Examples 16, 17, 18, 20A, or 20B.
Step 17: Apply the material selected from Step 16, for final coat. Applying only to one to two fingers at a time.
Step 18: Cure material applied in Step 17 for approximately one to approximately two minutes. Repeating Step 17 until all nails are finished.
Step 19: Remove residue from the coated nails with, for examples, alcohol or finishing compounds from Examples 3-6.
Step 20: Apply a solvent evaporation based high sheen coatings for nails if greater shine is desired.

Examples 27 through 32, described herein, give formulas for materials suitable for clear, French White Look, or colored radiation curable coatings. These examples optionally comprise clear or colored protective coatings on natural nails or artificial nails.

**Table 6. Examples 27 through 32 (quantities In grams)**

| Ingredient | 27 | 27A | 28 | 28A | 29 | 30 | 31 | 32 |
|---|---|---|---|---|---|---|---|---|
| BISGMA Urethane resin | -- | 0.17 | 3.0 | 3.0 | 1.94 | 2.95 | 109.7 | 1.82 |
| (meth)acrylated polyether urethane Resins | 5.72 | 0.92* | 15.74 | 5.28 | 9.41 | 15.50 | 602.8 | 9.56 |
| Ethyl toluenesulfonamide | 1.14 | 0.06 | 1.0 | 0.6 | 0.83 | 0.99 | 56.3 | 0.61 |
| Hydroxyethyl(meth)acrylate | 0.59 | -- | **--** | -- | 0.39 | -- | -- | -- |
| Benzildimethylketal | 0.0129 | 0.002 | 0.0400 | -- | -- | 0.0390 | 1.52 | 0.024 |
| Ethyl 2,4,6-trimethylbenzoyl-Phenylphosphinate | 0.0325 | 0.006 | 0.10 | -- | -- | 0.0986 | 4.0 | 0.061 |
| Trimethylolpropane tri(meth)acrylate | -- | -- | -- | -- | 0.10 | -- | -- | -- |
| Blend of 2,4,6-Trimethylbenzoyl-Diphenylphosphine-Oxide and methylbenzo-Phenone derivatives | -- | -- | -- | -- | 0.0634 | -- | -- | -- |
| 1-[4-(4-benzoylphenylsulfanyl)phenyl]-2-methyl-2-(4-methylphenylsulfanyl)propan-1-one | -- | -- | -- | 0.05 | -- | -- | -- | -- |
| Neoalkoxy titanate | 0.0065 | 0.002 | 0.0200 | 0.01 | 0.0320 | 0.0205 | 0.8 | 0.019 |
| Mono(methacryloyl-Oxyethyl) phthalate | -- | 0.035 | -- | -- | 0.54 | 0.59 | 24.8 | 0.37 |
| Dimethylaminoethyl(meth)acrylate | 0.0330 | 0.006 | 0.10 | 0.05 | 0.0609 | 0.0986 | 4.0 | 0.061 |
| Hydrogenated Rosin | -- | 0.093 | -- | -- | -- | 0.33 | -- | 0.52 |
| Ethyl Acetate | -- | 0.206 | -- | -- | -- | 0.16 | -- | 1.20 |
| D & C Red #7 Calcium Lake | -- | -- | -- | -- | 0.0011 | 0.1271 | -- | -- |
| D & C Red #6 Barium Lake | -- | 0.012 | -- | -- | -- | 0.0218 | -- | -- |
| Yellow #10 | -- | 0.001 | -- | -- | -- | -- | -- | -- |
| D&C Red #17 | -- | 0.001 | -- | -- | -- | -- | -- | -- |
| D & C Violet #2 | -- | 0.001 | -- | -- | -- | 0.0107 | -- | -- |
| Titanium dioxide coated mica platelets | -- | 0.001 | -- | -- | 0.0740 | -- | -- | -- |
| Iron oxide and mica | -- | -- | -- | -- | 0.0125 | -- | -- | -- |
| Titanium dioxide | -- | -- | -- | -- | 0.0409 | 0.0426 | -- | 0.1014 |

| | | | | | | | | |
|---|---|---|---|---|---|---|---|---|
| * Blend of three molecular weight components | | | | | | | | |

### Method of Creating a Temporary Natural Nail Enhancement with Color

The following method describes the use of formulas of Examples 22 through 31 to create a temporary natural nail enhancement with color that can be soaked off:
Step 1: File natural nail with a 220 grit file, to remove oil and shine and to minimize any subsequent lifting.
Step 2: Apply nail dehydrator (drying agent) to swab or nail wipe.
Step 3: Wipe nails with dehydrator to remove dust and minimize lifting.
Step 4: Apply a formaldehyde or non-formaldehyde traditional solvent evaporation base coat or a bond agent, for example, from one of the formulas presented In Examples 1-2A or 22-26 to natural nail and allow to dry for two minutes.
Step 5: Apply nail forms to the fingers under the free edge of the nail if desired to lengthen natural nails.
Step 6: Select a formula of Examples 27, 28, 28A,or 31.
Step 7: A small amount of the selected formula is applied to one side of a nail brush and then brush close to the cuticle to create a straight even line. The rest of the formula is brought down to the free edge and past it to cover and extend the nail.
Step 8: Cure the coating using UV radiation for approximately 2 minutes.
Step 9: Remove the nail forms.
Step 10: Remove any tackiness from the coated nails with, for example, alcohol.
Step 11: File and/or buff the nail into a desired shape.
Step 12: Remove dust.
Step 13: Select a color formula of Examples 27A, 29, 30, or 32.
Step 18: Polish the selected formula onto the entire surface.
Step 19: Cured the polished formula for approximately two minutes using UV radiation.
Step 20: Remove any tackiness from the polished nail surface using, for example, alcohol or finishing compounds from Examples 3-6.
Step 21: Apply a solvent evaporation based high sheen coatings for nails if greater shine is desired.

### Method of Creating a Temporary Natural Nail Enhancement with the French White Look

The following method describes the use of formulas of Examples 22 through 32 to create a temporary natural nail enhancement with the French White Look that can be soaked off:
Step 1: File natural nail with a 220 grit file, to remove oil and shine and to minimize any subsequent lifting.
Step 2: Apply nail dehydrator (drying agent) to swab or nail wipe.
Step 3: Wipe nails with dehydrator to remove dust and minimize lifting.
Step 4: Apply a formaldehyde or non-formaldehyde traditional solvent evaporation base coat or a bond agent, for example, from one of the formulas presented in Examples 1-2A or 22-26 to natural nail and allow to dry for two minutes.
Step 5: Apply nail forms to fingers under free edge of the natural nail.
Step 6: Select a formula from Example 32.
Step 7: Apply selected formula from Step 6 to free edge of natural nail. Preferably apply to one to two fingers at a time.
Step 8: Cure the applied material for approximately 2 minutes.
Step 9: Select a material from Examples 27, 28, 28A, 29, or 31.
Step 10: Apply selected material like polish on one to two fingers at a time.
Step 11: Cure material 2 minutes.
Step 12: Optionally repeat Steps 10 and 11 for additional coatings.
Step 13: Remove nail forms.
Step 14: Remove any tackiness from the coated nails with, for example, alcohol.
Step 15: Shape and buff cured material lightly with a 220 grit file, into a desired shape.
Step 16: Select a final coating material from Examples 27, 28, 28A, 29, or 31.
Step 17: Apply final coating material from step 16 on one to two nails at a time.
Step 18: Cure the applied material for approximately 2 minutes.
Step 19: Wipe residue from the coated nails with, for example, alcohol or finishing compounds from Examples 3-6.
Step 20: Apply a solvent evaporation based high sheen coatings for nails if greater shine is desired.

### Methods of Using Examples 22 through 32

The following method is applicable to any type of nail, the method as described in this embodiment, refers to toenails (in particular, the formula of Example 27A is preferred for toenail applications):
Step 1: Apply a nail dehydrator, for example, a drying agent to a cloth, wipe, etc.
Step 2: Wipe each toenail with the dehydrator to remove oil, lotion, etc.
Step 3: File each toenail to remove oil and shine and to minimize lifting.
Step 4: Apply a formaldehyde or non-formaldehyde traditional solvent evaporation base coat or a bond agent, for example, from one of the formulas presented in Examples 1-2A or 22-26 to natural nail and allow to dry for two minutes.
Step 5: Select a formula from, for example, one of the formulas presented in Examples 27 through 32.
Step 6: Apply the selected color formula, in a manner similar to application of a polish, to approximately one to approximately two toenails at a time; preferably apply the selected color formula on one side of a flat nail brush to allow for a closer approach to the cuticle; secure seal at free edge of natural nail to prevent lifting of product.
Step 7: Cure the applied formula for approximately 2 minutes.
Step 8: Repeat Steps 6 and 7 until all toenails are coated.
Step 9: Remove any residue from the coated toenails with, for example, alcohol or finishing compounds from Examples 3-6.
Step 10: Apply a solvent evaporation based high sheen coatings for nails if greater shine is desired.

### Method for Creating Permanent Nail Enhancements with Removable Color Coatings

This method describes the use of formulas of Examples 27 through 32 for removable color coatings on permanent artificial nails. For example, these formulas are useful in conjunction with nails prepared according to "Method of Creating a Permanent Nail Enhancement."
Step 1: A finished nail from the "Method of Creating a Permanent Nail Enhancement" is lightly buffed.
Step 2: Apply a formaldehyde or non-formaldehyde traditional solvent evaporation base coat.
Step 3: Allow the base coat to dry for two minutes.
Step 4: Select a color formula from, for example, one of the formulas presented in Examples 27A, 29, 30 or 32.
Step 5: The selected color gel is applied to a nail with a flat brush, starting close to the cuticle, creating a straight line around cuticle, and pulling down on rest of nail until all nail and free edge is covered with color.
Step 6: The applied gel coating is cured for approximately two minutes with UV radiation.
Step 7: An optional second coating of color is applied in the same fashion and cured for two minutes.
Step 8: Remove any residue from the coated nails with, for example, alcohol or finishing compounds from Examples 3-6.
Step 9: Apply a solvent evaporation based high sheen coating for nails if greater shine is desired.

The formulas presented In Examples 33 through 35 are useful In soaking off the coatings prepared by this aforementioned methods - "Method of Creating a Temporary Natural Nail Enhancement with Color", "Method of Creating a Temporary Natural Nail Enhancement with the French White Look", "Method of Using Examples 22 through 32", and "Method for Creating Permanent Nail Enhancements with Removable Color Coatings".

**Table 7. Examples 33 through 35 (quantities in grams).**

| Ingredient | 33 | 34 | 35 |
|---|---|---|---|
| Acetone | 7.09 | 6.38 | 6.86 |
| Squalene | 0.16 | 0.29 | 0.15 |
| Dipropylene Glycol Methyl ether | -- | 1.07 | -- |
| Lanolin | -- | -- | 0.72 |

### Methods of Using Examples 33 Through 35

The following embodiment describes a method for removing material comprising, for example, formulas from Examples 27 through 32, from toenails and/or fingernails:
Step 1: Select a soak-off agent from, for example, one of the formulas of Examples 33 through 35.
Step 2: Apply the selected soak-off agent to a carrier, for example, a cloth, wipe, etc.; preferably a wipe comprising approximately one square inch.
Step 3: Place the carrier comprising the selected soak-off agent onto a nail.
Step 4: Secure the carrier to the nail with, for example, a wrap, for example, but not limited to, a piece of aluminum comprising approximately 16 square Inches (e.g., approximately 4 inches square).
Step 5: Let the secured carrier set for approximately 10 minutes.
Step 6: Remove the secured carrier.
Step 7: Remove the soak-off agent treated material from the nail.

### Method of Lengthening or Improving the Appearance of Selected Nails

This method describes the use of formulas of Examples 27 through 32 with artificial nail tips to lengthen or improve the appearance of nails that may be shorter, hard to grow, broken, or damaged to create a balanced appearance of all ten nails:
Step 1: Buff the natural nail with a white block to remove the shine.
Step 2: Apply a nail tip and cut and blend into the natural nail.
Step 3: Apply a formaldehyde or non-formaldehyde traditional solvent evaporation base coat or a bond agent, for example, from one of the formulas presented in Examples 1-2A or 22-26 to natural nail and allow to dry for two minutes.
Step 5: Select a material from the formulas of Examples 27, 28, or 31.
Step 6: Apply the selected formula to the tip and natural nail.
Step 7: Cure the applied formula for approximately 1 to approximately 2 minutes using UV radiation.
Step 8: Optionally apply a second coat for more strength and/or a higher arch in the nail.
Step 9: Remove any tackiness with alcohol.
Step 10: File and/or buff the nail to a more desirable shape.
Step 11: Optionally top the nail with a clearcoat and/or colored coating selected from the formulas of Examples 27 through 32 and cure for two minutes.
Step 12: Remove any residue from the coated nails with, for example, alcohol or finishing compounds from Examples 3-6.
Step 13: Apply a solvent evaporation based high sheen coating for nails If greater shine is desired.

### Temporary Nail Tip and/or Sculpted Nail With Natural Nail Enhancements

Step 1: File natural nail with a 220-grit file, to remove oil and shine and to minimize any subsequent lifting.
Step 2: Apply a nail dehydrator (drying agent) to a swab or wipe.
Step 3: Wipe nails with dehydrator to remove dust and to minimize lifting.
Step 4: Select a nail tip and/or nail form for sculpting:
   Step 4a: Apply the nail tip or form
   Step 4b: If using a nail tip; cut the nail tip to a desired shape.
   Step 4c: Blend the nail tip to the natural nail.
Step 5: If using a nail form for sculpting, apply nail form to finger under free edge of the nail.
Step 6: Select a base coat, for example, from one of the formulas presented In the base coat agent examples mentioned above.
Step 7: Apply the base coat to the natural nail, which comprises a nail tip and/or nail form. Let dry 1 to 2 minutes.
Step 8: Select clear material, for example, from one of the formulas presented In the clear soak-off material examples given above.
Step 9: Apply the selected clear material like polish in one to two coats curing clear material between coasts for at least approximately 1 to approximately 2 minutes.
Step 10: Remove the nail forms.
Step 11: Remove any tackiness from the UV coated nails with, for example, alcohol.
Step 12: Lightly shape and buff cured material applied to the nail into a desired shape.
Step 13: Select a formula from Examples. These examples should include both color and clear material for soak off.
Step 14: Apply Example selected from Step 13, like polish on one to two nails at a time. Preferably, apply product on one side of a flat nailbrush. This allows for a closer approach to the cuticle. Secure seal at free edge of natural nail to prevent lifting. Remove any gel that on the skin or cuticle with a toothpick before curing.
Step 15: Cure the applied formula from Step 14 for 2 minutes.
Step 16: An optional second coating, from Example 14, is applied in the same fashion and cured 2 minutes.
Step 17: Remove any tackiness from nail with, for example, alcohol.
Step 18: Apply a solvent evaporation based high sheen coating for nails.

### Removal of Nail Tip or Sculpted Nail with Natural Nail Enhancements

Step 1: Select a soak off agent from, for example, one of the formulas of the soak-off agent examples, see above.
Step 2: Apply the selected soak off agent to a carrier, for example, a cloth, a wipe, etc. Preferably use a wipe comprising approximately one square inch.
Step 3: Place the carrier comprising the selected soak off agent onto a nail tip or sculpted nail.
Step 4: Secure the carrier to a nail tip or sculpted nail with a wrap, for example, but not limited to, a piece of aluminum comprising approximately 16 square inches (e.g. approximately 4 inches square).
Step 5: Let the secured carrier set for approximately 10 minutes.
Step 6: Remove the secured carrier.
Step 7: Remove any material that has loosened from the nail with, for example, a wipe, orange wood stick, or a nail file.
Step 8: Additional soaking can be applied to a nail to remove areas that have not loosened. Repeat steps 2 through 4, and let soak for only 5 minutes and remove the secured carrier.
Step 9: Remove the soak off agent treated material from the nail with, for example, a nail cleanser. Soak fingers in water for 3 to 5 minutes.

### Home Care Maintenance for Natural Nail Enhancements

Step 1: Buff nail with a buffing block to remove oils and shine and to minimize lifting.
Step 2: Apply a nail dehydrator (drying agent) to a swab or wipe.
Step 3: Wipe nails with dehydrator to remove dust and to minimize lifting.
Step 4: Select a color from one of the colored formula examples (this is preferably a soak clear or colored gel). The color formula selected should be the same or changed to a color staying close to color already existing on nails.
Step 5: From material selected in Step 4, start at cuticle area, fill gap that has grown out and brush down to cover entire nail. Preferably apply to one to two fingers at a time; preferably using a flat nailbrush and applying product to only one side of brush to allow for a closer approach to the cuticle.
Step 6: Cure the applied formula from Step 5 for 2 minutes.
Step 7: An optional second coating, from Step 4, is applied in the same fashion and cured 2 minutes.
Step 8: Remove any tackiness from nail with, for example, alcohol.
Step 9: Apply a solvent evaporation based high sheen coating for nails.

The Home Maintenance on Natural Nail Enhancements can be repeated up to three times before going to a salon professional.

### Other Methods within the Scope of the Present Invention

The aforementioned methods are applicable to the water-soluble and/or water-swellable base coatings disclosed In U.S. Patent No. 3,928,113, entitled "Method for coating human nails," to Rosenberg, issued December 23, 1975.

The preceding examples can be repeated with similar success by substituting the generically or specifically described reactants and/or operating conditions of this invention for those used in the preceding examples.

## Claims

1. Use of a composition comprising a suitable amount of a polymerizable polyol modified (meth)acrylate resin as a soak-off nail coating, wherein the composition is applied to a coated nail and cured.

2. The method according to claim 1, wherein said polymerizable polyol modified (meth)acrylate resin comprises a (meth)acrylate urethane resin.

3. The use according to claim 1 or 2, wherein said cured composition is removable or softenable with polar organic solvents.

4. Method of applying a soak-off nail coating composition to a coated nail, the method comprising the steps of:
providing a coated nail,
applying a soak-off nail coating composition comprising a suitable amount of a polymerizable polyol modified (meth)acrylate resin to the coated nail, and
curing the applied composition.

5. The method according to claim 4, wherein said polymerizable polyol modified (meth)acrylate resin comprises a (meth)acrylate urethane resin.

6. The method according to claim 4 or 5, wherein said composition is removable or softenable with polar organic solvents.

7. Method of removing a soak-off nail coating composition from a coated nail, the method comprising the steps of:
providing a nail coated with a radiation cured composition, the cured composition comprising a suitable amount of a polymerizable polyol modified (meth)acrylate resin; and
soaking the coated nail with a solvent, wherein the solvent comprises a polar solvent.

8. The method according to claim 7, wherein said polymerizable polyol modified (meth)acrylate resin comprises a (meth)acrylate urethane resin.

## Patentansprüche

1. Verwendung einer Zusammensetzung umfassend eine geeignete Menge eines polymerisierbaren, polyolmodifizierten (Meth)acrylatharzes als abweichbare Nagelbeschichtung, wobei die Zusammensetzung auf einen beschichteten Nagel aufgebracht und ausgehärtet wird.

2. Verfahren nach Anspruch 1, wobei das polymerisierbare, polyolmodifizierte (Meth)acrylatharz ein (Meth)acrylaturethanharz umfasst.

3. Verwendung nach Anspruch 1 oder 2, wobei die ausgehärtete Zusammensetzung mit polaren organischen Lösungsmitteln entfernbar oder erweichbar ist.

4. Verfahren zum Aufbringen einer abweichbaren Nagelbeschichtungszusammensetzung auf einen beschichteten Nagel, wobei das Verfahren die Schritte umfasst des:
Bereitstellens eines beschichteten Nagels,
Aufbringens einer abweichbaren Nagelbeschichtungszusammensetzung umfassend eine geeignete Menge eines polymerisierbaren, polyolmodifizierten (Meth)acrylatharzes, um den Nagel zu beschichten, und Aushärtens der aufgebrachten Zusammensetzung.

5. Verfahren nach Anspruch 4, wobei das polymerisierbare, polyolmodifizierte (Meth)acrylatharz ein (Meth)acrylaturethanharz umfasst.

6. Verfahren nach Anspruch 4 oder 5, wobei die Zusammensetzung mit polaren organischen Lösungsmitteln entfernbar oder erweichbar ist.

7. Verfahren zum Entfernen einer abweichbaren Nagelbeschichtungszusammensetzung von einem beschichteten Nagel, wobei das Verfahren die Schritte umfasst des:
Bereitstellens eines mit einer strahlungsausgehärteten Zusammensetzung beschichteten Nagels, wobei die ausgehärtete Zusammensetzung eine geeignete Menge eines polymerisierbaren, polyolmodifizierten (Meth)acrylatharzes umfasst; und
Einweichens des beschichteten Nagels in einem Lösungsmittel, wobei das Lösungsmittel ein polares Lösungsmittel umfasst.

8. Verfahren nach Anspruch 7, wobei das polymerisierbare, polyolmodifizierte (Meth)acrylatharz ein (Meth)acrylaturethanharz umfasst.

## Revendications

1. Utilisation d'une composition comprenant une quantité adaptée d'une résine de (méth)acrylate modifiée par un polyol polymérisable en tant que revêtement d'ongles soak-off, la composition étant appliquée sur un ongle revêtu et durci.

2. Procédé selon la revendication 1, ladite résine de (méth)acrylate modifiée par un polyol polymérisable comprenant une résine de (méth)acrylate-uréthane.

3. Utilisation selon la revendication 1 ou 2, ladite composition durcie pouvant être retirée ou ramollie avec des solvants organiques polaires.

4. Procédé d'application d'un revêtement d'ongles soak-off sur un ongle revêtu, le procédé comprenant les étapes consistant à :
fournir un ongle revêtu,
appliquer une composition de revêtement d'ongles soak-off comprenant une quantité adaptée d'une résine de (méth)acrylate modifiée par un polyol polymérisable sur l'ongle revêtu, et durcir la composition appliquée.

5. Procédé selon la revendication 4, ladite résine de (méth)acrylate modifiée par un polyol polymérisable comprenant une résine de (méth)acrylate-uréthane.

6. Procédé selon la revendication 4 ou 5, ladite composition pouvant être retirée ou ramollie avec des solvants organiques polaires.

7. Procédé de retrait d'une composition de revêtement d'ongles soak-off d'un ongle revêtu, le procédé comprenant les étapes consistant à :
fournir un ongle revêtu avec une composition durcie par rayonnement, la composition durcie comprenant une quantité adaptée d'une résine de (méth)acrylate modifiée par un polyol polymérisable ; et tremper l'ongle revêtu avec un solvant, le solvant comprenant un solvant polaire.

8. Procédé selon la revendication 7, ladite résine de (méth)acrylate modifiée par un polyol polymérisable comprenant une résine de (méth)acrylate-uréthane.
